# EUROPÄISCHE PATENTANMELDUNG

(11) **EP 1 334 961 A1**
(43) Veröffentlichungstag der Anmeldung: **13.08.2003**
(21) Anmeldenummer: 03008611.0
(22) Anmeldetag: 07.06.1994
(51) Int. Cl.: C07C 227/00, C07C 229/16, C07C 255/25

(54) **Verfahren zur Herstellung von Glycin-N,N-diessigsäure-Derivaten**

(30) Priorität: 16.06.1993 DE 4319935
(62) Teilanmeldung aus: 97101598.7
(71) Anmelder: BASF AKTIENGESELLSCHAFT, 67056 Ludwigshafen (DE)
(72) Erfinder: Schneider, Jürgen, Dr., 67273 Bobenheim am Berg (DE); Potthoff-Karl, Birgit, Dr., 67061 Ludwigshafen (DE); Kud, Alexander, Dr., 55234 Eppelslheim (DE); Baur, Richard, Dr., 67112 Mutterstadt (DE); Oftring, Alfred, Dr., 67098 Bad Dürkheim (DE); Greindl, Thomas, Dr., 67098 Bad Dürkheim (DE)

(57) **Zusammenfassung**

Herstellung von Glycin-N,N-diessigsäure-Derivaten I 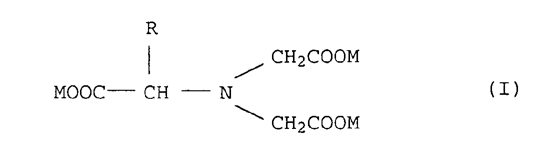 wobei
- R: für Methyl steht und
- M: Wasserstoff, Alkalimetall, Erdalkalimetall, Ammonium oder substituiertes Ammonium in den entsprechenden stöchiometrischen Mengen bedeutet,
indem man entsprechende 2-substituierte Glycine oder 2-substituierte Glycinnitrile mit Formaldehyd und Cyanwasserstoff oder Alkalimetallcyanid umsetzt und anschließend noch vorhandene Nitrilgruppen zu Carboxylgruppen hydrolysiert.

## Beschreibung

Die vorliegende Erfindung betrifft ein Verfahren zur Herstellung von Glycin-N,N-diessigsäure-Derivaten und bei diesem Verfahren auftretende Zwischenprodukte.

Die DE-A 37 12 329 offenbart ein Verfahren zur Herstellung von Serin-N,N-diessigsäurederivaten durch Umsetzung eines hydroxymethylsubstituierten Glycins mit Formaldehyd und Cyanwasserstoff.

Aufgabe der vorliegenden Erfindung war es, ein Verfahren zur Herstelung von Glycin-N,N-diessigsäure-Derivaten bereitzustellen, welche die Nachteile des Standes der Technik nicht mehr aufweisen.

Demgemäß wurde ein Verfahren zur Herstellung von Glycin-N,N-diessigsäure-Derivaten der allgemeinen Formel I 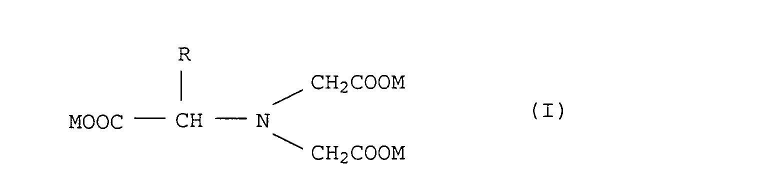 in der
- R: für Methyl steht und
- M: für Wasserstoff, Alkalimetall, Erdalkalimetall, Ammonium oder substituiertes Ammonium in den entsprechenden stöchiometrischen Mengen bedeutet,
gefunden, welches dadurch gekennzeichnet ist, daß man entsprechende 2-substituierte Glycine oder 2-substituierte Glycinnitrile mit Formaldehyd und Cyanwasserstoff oder Alkalimetallcyanid umsetzt und anschließend noch vorhandene Nitrilgruppen zu Carboxylgruppe hydrolysiert.

Die genannte Ausführungsform stellt ein Beispiel für die "Strekker-Synthese" dar, bei der allgemein Aldehyde mit Ammoniak oder Aminen und Blausäure ("saure" Variante) oder Cyaniden ("alikalische" Variante) zu Aminosäuren oder Derivate hiervon umgesetzt werden.

Die "alkalische" Variante der Strecker-Synthese wird beispielsweise in der US-A 3 733 355 in allgemeiner Form dargestellt. Die dort aufgeführten Beispiele zeigen jedoch, daß immer ein hoher Anteil an Nebenprodukten,- vor allem an unerwünschter Glykolsäure, auftritt; dies läßt sich aus den Umsätzen von nur maximal ca. 89 % schließen.

Die "saure" Variante der Strecker-Synthese ist beispielsweise aus der DE-A 20 27 972 bekannt. Dort wird die Herstellung von Carboxymethyliminodiacetonitril ausgehend von Glycin, Formaldehyd und Blausäure in saurem Medium beschrieben. Hierbei wird empfohlen, zusätzliche Säure zuzugeben, um den pH-Wert im Bereich unterhalb von 7 zu halten.

Aufgabe der vorliegenden Erfindung war es auch, ein effektiveres und wirtschaftlicheres Verfahren zur Herstellung von Glycin-N,N-diessigsäure-Derivaten I bereitzustellen, das insbesondere die Bildung von unerwünschten Nebenprodukten unterdrückt und auf zusätzliche Hilfsstoffe, beispielsweise zur pH-Wert-Regulierung, verzichten kann.

Als besonders vorteilhaft haben sich die Varianten herausgestellt, bei denen mit Cyanwasserstoff ("saure" Variante) gearbeitet wird. Zweckmäßigerweise setzt man wasserfreien Cyanwasserstoff ein, der großtechnisch üblicherweise in dieser Form gehandhabt wird.

Die Umsetzung wird bevorzugt in Wasser, aber auch in einem organischen Lösungsmittel oder in Mischungen hieraus durchgeführt. Als organische Lösungsmittel kommen vorzugsweise solche zum Einsatz, die mit Wasser teilweise oder vollständig mischbar sind, z. B. Methanol, Ethanol, n-Propanol, iso-Propanol, tert.-Butanol, Dioxan oder Tetrahydrofuran. Auch Lösungsvermittler können verwendet werden.

Man setzt zweckmäßigerweise pro Mol Aminoverbindung 2 bis 2,6 mol Formaldehyd, vorzugsweise in Form seiner wäßrigen ca. 30 gew.-%igen Lösung, und 2 bis 2,3 mol Cyanwasserstoff oder Alkalimetallcyanid, beispielsweise Natrium- oder Kaliumcyanid, ein. Die Umsetzung wird normalerweise im Falle von wasserfreiem Cyanwasserstoff bei Temperaturen von 0 bis 120°C, insbesondere 15 bis 80°C, und im Falle von Alkalimetallcyaniden bei 40 bis 110°C, insbesondere 70 bis 100°C, vorgenommen.

An diese Umsetzung schließt sich eine Hydrolyse noch vorhandener Nitrilgruppen zu Carboxylgruppen an, welche in an sich bekannter Weise in wäßrigem Reaktionsmedium in Gegenwart von Basen wie Natron- oder Kalilauge oder von Säuren wie Schwefel- oder Salzsäure bei Temperaturen von 20 bis 110°C, insbesondere 40 bis 100°C, durchgeführt wird.

Die als Ausgangs-Aminoverbindungen verwendeten Glycin- und Glycinnitril-Derivate können sowohl als Racemate als auch als enantiomerenreine D- oder L-Verbindungen eingesetzt werden.

Entsprechend den Reaktionsbedingungen erhält man die Glycin-N,N-diessigsäure-Derivate I als freie Carbonsäure oder beispielsweise als Alkalimetallsalz. Aus der freien Säure können durch Neutralisation mit den entsprechenden Basen, beispielsweise Aminbasen, die gewünschten Salze ohne Schwierigkeit hergestellt werden.

Die Glycin-N,N-diessigsäure-Derivate I und ihre Salze lassen sich aus ihren Lösungen problemlos in reiner Form isolieren. Hierfür bieten sich insbesondere Sprüh- oder Gefriertrocknung, Kristallisation und Fällung an. Es kann vorteilhaft sein, die bei der Herstellung angefallene Lösung direkt einer technischen Verwendung zuzuführen.

Gegenstand der vorliegenden Erfindung sind auch die in der Literatur noch nicht bekannten in 2-Position durch den Rest R substituierten Glycin-N,N-diacetonitrile und Glycinnitril-N,N-diacetonitrile, bei denen R für Methyl steht, also die Verbindungen α-Alanin-N,N-diacetonitril und α-Alaninnitril-N,N-diacetonitril, als Zwischenprodukte für die Herstellung von Glycin-N,N-diessigsäure-Derivaten I und ihren Salzen. Diese Verbindungen entstehen als Zwischenstufen bei der Umsetzung der genannten Glycin- bzw. Glycinnitril-Derivate mit Formaldehyd und Cyanwasserstoff

Beim erfindungsgemäßen Verfahren läßt sich bei der "sauren" Variante mit in der 2-Position substituierten Glycinen als Ausgangsmaterial auf zusätzliche Säure verzichten, da erstaunlicherweise die Acidität der vorhandenen Carboxylgruppe für die Durchführung der Umsetzung ausreichend ist.

Generell erhält man das Umsetzungsprodukt in hoher Ausbeute in ausreichend reiner Form. Der Gehalt an Nebenprodukten ist gering. Weitere Vorteile des erfindungsgemäßen Herstellungsverfahrens sind die salzfreie Fahrweise und die leicht verfügbaren Einsatzstoffe.

Herstellungsbeispiel

### Beispiel 2

### Herstellung von α-D,L-Alanin-N,N-diessigsäure-Trinatriumsalz aus α-D,L-Alanin

Zu einer Suspension von 44 g D,L-Alanin (> 99 gew.-%ig) in 200 g Wasser gab man bei 30°C gleichzeitig 105 g Formaldehyd (30 gew.-%ig) und 31,7 g Blausäure (89,5 gew.-%ig). Es wurde noch 3 Stunden bei 30°C nachgerührt. Die Blausäure-Abnahme entsprach einem Umsatz von > 97 % der Theorie.

Die so erhaltene wäßrige Lösung von α-D,L-Alanin-N,N-diacetonitril wurde bei 30°C zu 132 g 50 gew.-%iger Natronlauge getropft. Nach 8-stündigem Rühren bei 30°C wurde die Temperatur auf 95 bis 102°C erhöht. Nach weiteren 4 Stunden war die Umsetzung praktisch vollständig. Man erhielt 352,5 g einer Lösung, die gemäß ihrem Eisen-Bindevermögen 37,4 Gew.-% α-D,L-Alanin-N,N-diessigsäure-Trinatriumsalz enthielt (entsprechend einer Ausbeute von 97,4 % der Theorie über beide Stufen).

## Patentansprüche

1. Verfahren zur Herstellung von Glycin-N,N-diessigsäure-Derivaten der allgemeinen Formel I 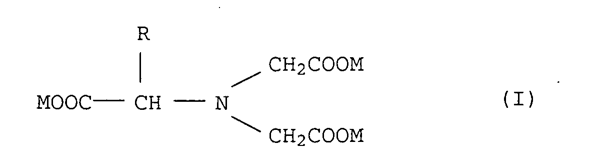 in der
R für Methyl, steht und
M Wasserstoff, Alkalimetall, Erdalkalimetall, Ammonium oder substituiertes Ammonium in den entsprechenden stöchiometrischen Mengen bedeutet,
**dadurch gekennzeichnet, daß** man entsprechende 2-substituierte Glycine oder 2-substituierte Glycinnitrile mit Formaldehyd und Cyanwasserstoff oder Alkalimetallcyanid umsetzt und anschließend noch vorhandene Nitrilgruppen zu Carboxylgruppen hydrolysiert.

2. Verfahren zur Herstellung von Glycin-N,N-diessigsäure-Derivaten I nach Anspruch 1, **dadurch gekennzeichnet, daß** man auf die Mitverwendung zusätzlicher Säure verzichtet.

3. In 2-Position durch den Rest R substituierte Glycin-N,N-diacetonitrile und Glycinnitril-N,N-diacetonitrile, bei denen R für Methyl steht, als Zwischenprodukte.
